# EUROPEAN PATENT SPECIFICATION

(11) **EP 1 597 807 B1**
(45) Date of publication and mention of the grant of the patent: **25.04.2007**
(21) Application number: 04710622.4
(22) Date of filing: 12.02.2004
(51) Int. Cl.: H02J 7/00, H02K 7/18, A61N 1/378, A61N 1/39

(54) **SELF-POWERED IMPLANTABLE ELEMENT**
IMPLANTIERBARE VORRICHTUNG MIT EIGENER ENERGIEVERSORGUNG
ELEMENT IMPLANTABLE A AUTO-ALIMENTATION

(30) Priority: 12.02.2003 US 365893
(43) Date of publication of application: 23.11.2005
(73) Proprietor: Medtronic Inc., Minneapolis, MN 55432 (US)
(72) Inventor: THOMPSON, David, L., Andover, Minnesota 55304 (US)
(74) Representative: Hughes, Andrea Michelle
(86) International application number: PCT/US2004/004045
(87) International publication number: WO 2004/073138

(56) References cited:
- EP-A- 0 974 377
- US-A- 4 798 206
- US-A- 5 431 694
- US-A- 5 540 729
- US-A- 5 941 904

## Description

The invention relates to implantable medical devices, and more particularly, to implantable medical devices that include a microelectromechanical system (MEMS).

An implantable medical device (IMD) may receive signals from or transmit signals to one or more elements such as sensors. A sensor may be responsive to a sensed condition in the body, such as electrical activity, blood pressure, blood chemistry or a mechanical property. Sensors responsive to sensed conditions may detect or measure a quantity of clinical significance.

An IMD may also communicate with an element that may provide one or more therapeutic functions. For example, an electrode may deliver a therapeutic electric shock to nearby tissue. Some elements may perform both sensing and therapeutic functions. Many elements require a source of power to function or to transmit signals to an IMD. Conventional sensors, for example, may receive power from a dedicated battery, or from the IMD, which typically includes a battery. An IMD conventionally delivers power to a sensor by way of a lead that includes a conductor.

In general, the invention is directed to elements that receive power from an implantable motion-powered energy source. A motion-powered energy source converts mechanical energy in the form of motion into electrical energy. As the motion-powered energy source moves, the motion-powered energy source generates electrical energy that may be stored in an energy storage device such as a capacitor. This electrical energy may power the element.

A microelectromechanical systems (MEMS) accelerometer may be used to generate electrical energy in response to motion. A MEMS accelerometer may include one or more capacitive elements that change geometry when the MEMS accelerometer is subjected to changes in motion. In an embodiment of the invention, the MEMS accelerometer may be employed proximate to cardiac tissue, and may be bobbed, jiggled, rocked, or twisted by the natural motion of the beating heart. The MEMS accelerometer converts a portion of this motion into electrical energy, which may be stored and used to power an element.

The invention is directed to a device comprising:
a generator implantable in a human body to convert mechanical energy into electrical energy;
an energy storage device to receive the electrical energy from the generator and to store the electrical energy; and
an element implantable in the human body that receives energy from the energy storage device; characterised in that said generator comprises a microelectromechanical systems (MEMS) accelerometer comprising one or more capacitive elements whose geometry changes in response to movement of an organ of the human body in proximity to which the generator is, in use, implanted, causing a change in the capacitance of the accelerometer. The generator may include one or more MEMS accelerometers of different configurations.

The invention may offer one or more inventive aspects. An element powered by a motion-powered energy source need not rely on a dedicated battery or on energy from an IMD. As a result, the number of electrical connections between the IMD and the element may be reduced. In some cases, all physical connection between the element and the IMD may be eliminated. Instead, the element and IMD may be configured for wireless communication. With fewer connections, lead construction may be simplified, and the lead and the element may be more robust.

In addition, the invention may result in a saving of space. The element need not have a bulky battery, allowing the element to be more miniaturized and more versatile. The motion-powered energy source also is small and saves space.
Furthermore, a motion-powered energy source does not run down like a battery, but may generate power for an indefinite period of time. The motion-powered energy source may be placed proximate to an organ that moves, such as the gastrointestinal system or diaphragm, and may generate power from the motion of the organ. When placed proximate to a heart so that the motion-powered energy source moves with each beat of a heart, the motion-powered energy source will continue to generate power as long as the heart continues to beat.

The details of one or more embodiments of the invention are set forth in the accompanying drawings and the description below. Other features, aspects and significant elements of the invention will be apparent from the description and drawings, and from the claims.
FIG. 1 is a schematic view of a human heart with an implantable medical device and a lead extending from the implantable medical device to the heart.
FIG. 2 is a cross-sectional side view of an embodiment of the distal end of the lead shown in FIG. 1.
FIG. 3 is a block diagram illustrating an exemplary implementation of motion-powered energy source that supplies power to an element.
FIG. 4 is a circuit diagram that models an implementation of a motion-powered energy source.
FIG. 5 is another circuit diagram that models another implementation of a motion-powered energy source.

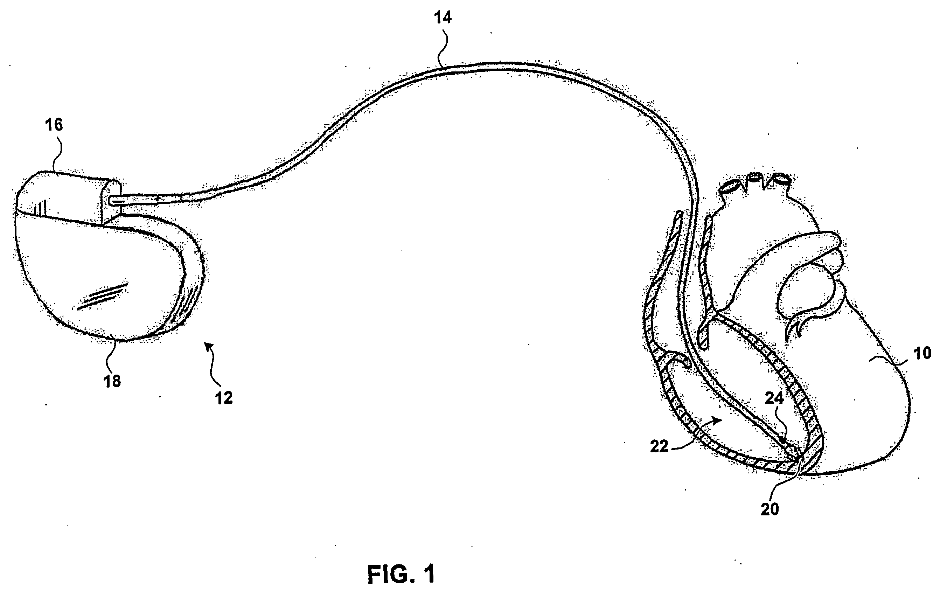

FIG. 1 is a schematic view showing a human heart 10 of a patient, with an implantable medical device (IMD) 12. IMD 12 shown in FIG. 1 is a pacemaker comprising a pacing and sensing lead 14. Lead 14 comprises an elongated lead body with a proximal end and a distal end. The proximal end of lead 14 is attached to connector module 16 of a hermetically sealed enclosure 18. An electrode 20 at the distal end of lead 14 is disposed in the right ventricle 22 of heart 10. IMD 12 senses electrical signals attendant to the depolarization and repolarization of heart 10 via electrode 20 and lead 14. IMD 12 further generates pacing pulses that are delivered to electrode 20 via lead 14, and that cause depolarization of cardiac tissue in the vicinity of electrode 20.

A sensor 24 is disposed proximate to the distal end of lead 14, and is disposed in right ventricle 22. Sensor 24 may be an element that detects or measures a physiological quantity of clinical significance. For example, sensor 24 may be a pressure sensor responsive to blood pressure inside right ventricle 22, or an oxygen sensor responsive to the oxygen concentration in the blood. Sensor 24 may be a pH sensor or a glucose sensor or other sensor responsive to blood chemistry. Sensor 24 may also include a device responsive to the activity or mechanical properties of the organ, such as a strain gauge, motion sensor or contractility sensor.

Although the invention is described in terms of a sensor, the invention also includes embodiments with elements other than sensors. The invention includes embodiments that comprise an element that delivers therapy, or an element that performs therapeutic and sensing functions. An electrode that can detect electrical activity and deliver therapeutic electric shocks is an example of an element that may perform therapeutic and sensing functions. The invention may also be practiced with an element that controls therapy administered by another medical device, such as a neurostimulation device or an element that controls delivery of a drug or other therapeutic substance from an implanted reservoir.

Sensor 24, like other elements, may require a source of power. Sensor 24 may require power to detect or measure the sensed conditions. Sensor 24 may also require power to transmit a signal to IMD 12 indicative of the response of sensor 24 to the sensed condition. Other functions of sensor 24 may also require power.
Sensor 24 receives power from a motion-powered energy source (not shown) proximate to sensor 24. The motion-powered energy source converts mechanical energy, i.e., motion, into electrical energy, and stores that energy in an energy storage device. Sensor 24 receives and uses energy from the energy storage device. In effect, the motion-powered energy source behaves like an implantable electrical generator that powers sensor 24.
In the embodiment shown in FIG. 1, the motion-powered energy source may be disposed at the distal end of lead 14. As heart 10 beats, the distal end of lead 14 moves. Moreover, the magnitude and direction of the motion are not constant. For example, the distal end of lead 14 may bob, jiggle, rock, and twist as right ventricle 22 contracts and relaxes. In addition, the distal end of lead 14 may move due to the motion of the patient. The motion-powered energy source converts this mechanical energy into electrical energy, which is used to power sensor 24.

IMD 12 shown in FIG. 1 is exemplary, and the invention is not limited to the application shown. Rather, the invention may be practiced with implantable medical devices that provide a variety of pacing or other therapies, such as cardioversion or defibrillation. Although FIG. I depicts a single-chamber pacemaker with a unipolar lead, the invention may be practiced by multi-chamber devices and by devices with bipolar leads. The invention may also be practiced with an implantable medical device that provides no therapy at all, such as a heart monitor. The invention may further be practiced with other implantable medical devices, such as loop recorders, neurological stimulation devices or implantable drug delivery systems. The implantable medical devices need not be proximate to heart 10.

Moreover, the invention is not limited to implantable medical devices proximate to heart 10, but may be practiced with devices implanted to monitor or provide therapy to other organs. There may be an advantage to disposing the motion-powered energy source in or near heart 10, however, because of the repetitive motion of heart 10. The repetitive motion 10 provides the motion-powered energy source with a substantially constant supply of mechanical energy, which can be harnessed to generate electrical energy. Other organs or systems, such as the gastrointestinal system, diaphragm or esophagus, likewise may provide sources of mechanical energy.

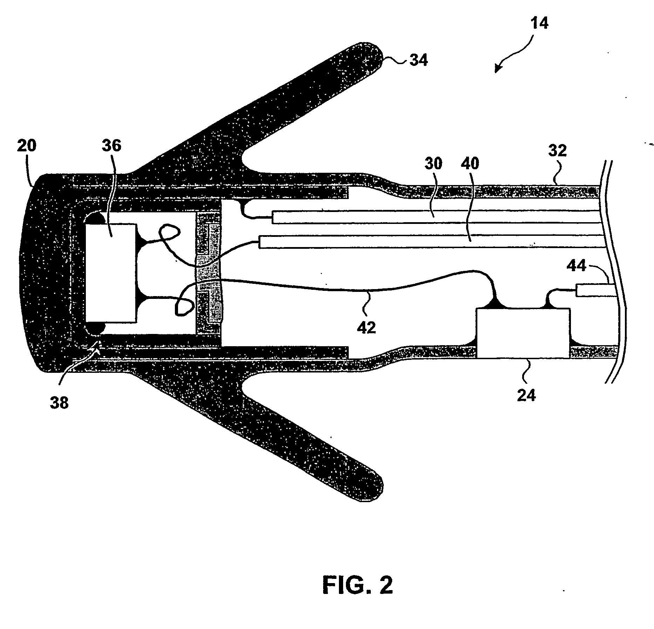

FIG. 2 is a cross-sectional side view of an embodiment of the distal end of lead 14. The distal end of lead 14 includes electrode 20, which transmits or receives electrical signals or pacing stimuli from IMD 12 (not shown in FIG. 2) via a conductor 30. Electrode 20 is coupled to an insulating sheath 32. Tines 34 projecting from sheath 32 present a fixation mechanism that anchors the distal end of lead 14 in cardiac tissue. A motion-powered energy source 36 is disposed inside the distal end of lead 14. As shown in FIG. 1, motion-powered energy source 36 may be housed inside a capsule 38 and may be electrically coupled to IMD 12 via one or more conductors 40. Conductor 40 may, for example, supply a voltage potential to motion-powered energy source 36 or may transmit signals between IMD 12 and motion-powered energy source 36. Sensor 24 is electrically coupled to motion-powered energy source 36 via conductor 42. Motion-powered energy source 36 converts mechanical energy, including energy in the form of motion of heart 10, into electrical energy. Motion-powered energy source 36 stores that energy in an energy storage device. Sensor 24 receives energy from the energy storage device via conductor 42. In this way, motion-powered energy source 36 powers sensor 24.

Sensor 24 may apply power from motion-powered energy source 36 to detect or measure sensed conditions. In some embodiments, sensor 24 may also apply power from motion-powered energy source 36 to deliver an electrical shock to adjacent tissue. In further embodiments, sensor 24 may apply power from motion-powered energy source 36 to transmit signals to IMD 12 via conductor 44.

The arrangement depicted in FIG. 2 is exemplary, and the invention is not limited to the application shown. Motion-powered energy source 36 need not be housed in a capsule, and need not be electrically coupled to IMD 12 by a conductor. In addition, the invention may be practiced with leads of various configurations, including leads with bipolar electrodes, leads with fixation mechanisms other than tines, or leads configured to provide steroid elution.

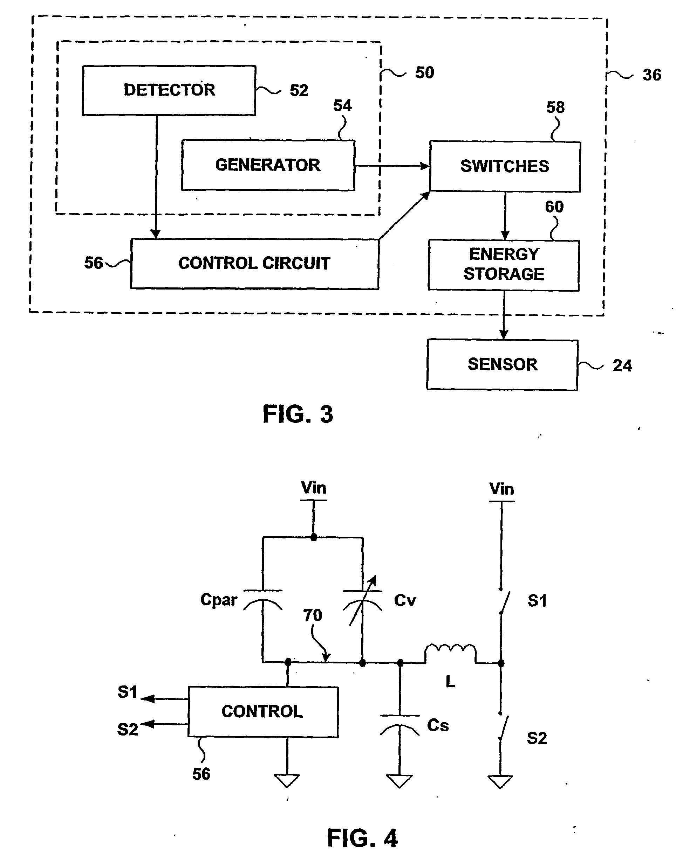

FIG. 3 is a block diagram illustrating an example implementation of motion-powered energy source 36. Motion-powered energy source 36 includes a microelectromechanical systems (MEMS) accelerometer 50. MEMS accelerometer 50 includes capacitive elements that respond to motion by changing the geometry of the capacitive elements, which results in changes in capacitance. In general, the capacitance is a function of the distance between charged structures. In MEMS accelerometer 50, motion of the device causes the capacitance of the device to change. In particular, changes in the magnitude or direction of the velocity of the device cause changes in one or more distances among components, thereby changing the capacitance.

In general, a MEMS device integrates mechanical and electrical components on a substrate through microfabrication and micromachining techniques. A MEMS accelerometer comprises a mass suspended on the surface of an integrated circuit. In response to motion, the mass may move relative to other structures on the MEMS accelerometer, thereby changing the geometry of the device as a whole. In many MEMS accelerometers, the mass is spring-mounted or otherwise biased to return to an equilibrium position. As a result, the mass may vibrate in response to motion.

MEMS accelerometer 50 may comprise any of several configurations. In an in-plane overlap MEMS accelerometer, the MEMS accelerometer typically includes a plurality of interdigitated projecting structures or "fingers," sometimes arranged in "combs," with the area of overlap of fingers being variable in response to motion. As the overlap area changes, the capacitance of the in-plane overlap MEMS accelerometer changes. An in-plane gap-closing MEMS accelerometer likewise includes fingers, with the capacitance changing as the gap between the fingers changes. The capacitance of an out-of-plane gap-closing MEMS accelerometer changes as a function of the size of a variable gap between two plates.

The invention may be practiced with any of an in-plane overlap MEMS accelerometer, an in-plane gap-closing MEMS accelerometer or an out-of-plane gap-closing MEMS accelerometer. The invention is not limited to these configurations, however.

The changes in capacitance due to motion of MEMS accelerometer 50 result in voltage generation. The capacitance of a capacitor (C), the charge stored in the capacitor (Q), and the voltage across the capacitor (V) are related by the equation Q = CV, or V = Q/C. Given a fixed charge, the voltage across the capacitor varies inversely with the capacitance. Thus, as the capacitance of MEMS accelerometer 50 changes with motion, the voltage across MEMS accelerometer 50 changes as well.

In FIG. 3, a small section of MEMS accelerometer 50 operates as a detector 52, and detects the changing capacitance of MEMS accelerometer 50 under vibration. The balance of MEMS accelerometer 50 operates as a generator 54, which generates a variable voltage in response to motion. A control circuit 56, in response to signals from detector 52, controls switches 58 to transfer energy from generator 54 to an energy storage device 60.

Control circuit 56 may comprise an integrated circuit including analog or digital signal processing elements. In some embodiments of the invention, control circuit 56 may be embodied as a dedicated processor, such as a field-programmable gate array. In other embodiments of the invention, control circuit 56 may be embodied as a general purpose microprocessor.

Switches 58 may be any of several semiconductor devices having on and off modes. Switches 58 may comprise, for example, field effect transistors, bipolar junction transistors, diodes, or any combination thereof.

In response to motion, the capacitance of MEMS accelerometer 50 oscillates between high and low values. Accordingly, the voltage across MEMS accelerometer 50 oscillates between low and high values as a function of the varying capacitance. Control circuit 56 uses signals from detector 52 to determine whether the voltage across MEMS accelerometer 50 is low or high. When the voltage across MEMS accelerometer 50 is high, control circuit 56 controls switches 58 to store energy in energy storage device 60.

Energy may be stored in energy storage device 60 incrementally. Over time, energy storage device 60 may store a substantial quantity of energy. Energy storage device 60 supplies the stored energy to sensor 24, thereby powering sensor 24. Energy storage device 60 may comprise a storage capacitor. In some embodiments of the invention, energy storage device 60 may include additional components to pump energy into the storage capacitor, thereby incrementally increasing the energy stored by energy storage device 60.

FIG. 4 is a circuit diagram of a model illustrating an implementation of motion-powered energy source 36. MEMS accelerometer 50 is represented by a variable capacitor Cv, with a parasitic capacitance Cpar. With one terminal of capacitor Cv held at a fixed voltage by supply voltage Vin, the node voltage at the opposite terminal 70 is free to increase or decrease, in response to changes in Cv.

When the voltage at node 70 drops or rises, control circuit 56 opens and closes switches S1 or S2 to cause some energy stored in variable capacitor Cv to be stored in storage capacitor Cs. Storage capacitor Cs, inductor L and switches S1 and S2 cooperate to form a resonant charge pump that stores energy in storage capacitor Cs, thereby increasing the voltage across capacitor Cs. The voltage across storage capacitor Cs may be supplied to sensor 24 (not shown in FIG. 4), thereby powering sensor 24.

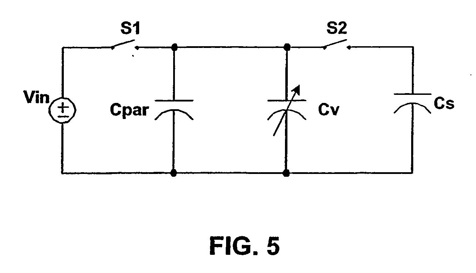

FIG. 5 is a circuit diagram of an alternate model illustrating an implementation of motion-powered energy source 36. Once again, MEMS accelerometer 50 is represented by a variable capacitor Cv, with a parasitic capacitance Cpar. As the capacitance of variable capacitor Cv changes, switches S1 and S2 open and close to store energy in storage capacitor Cs. Switches S1 and S2 may be controlled by control circuit 56 (not shown in Fig. 5) or may be diodes that turn on or off according to voltages and currents in the circuit.

When the capacitance of variable capacitor Cv declines from a peak, switches S 1 and S2 are open. As the capacitance declines, the voltage across variable capacitor Cv rises. When the voltage across variable capacitor Cv is sufficiently high, switch S2 closes and charge flows from variable capacitor Cv to storage capacitor Cs. At this stage, the total charge in the circuit is constant, but the falling capacitance of variable capacitor Cv causes the voltage across variable capacitor Cv and the voltage across storage capacitor Cs to increase. At this stage, therefore, mechanical energy is converted to electrical energy. When the capacitance of variable capacitor Cv reaches a minimum value and the voltage across variable capacitor Cv reaches a high value, switches S1 and S2 are open. As the capacitance rises, the voltage across variable capacitor Cv falls. When the voltage across variable capacitor Cv is sufficiently low, switch S1 closes and charge flows from a voltage source Vin to variable capacitor Cv. The cycle may repeat. By repetition of the cycle, charge may be pumped to storage capacitor Cs, increasing the voltage across storage capacitor Cs.

The amount of energy generated using the above techniques depends upon the configuration and size of MEMS accelerometer that is used. It is believed that some embodiments may generate fifty microwatts per square centimeter. This generation capacity could support any of a number of implantable elements, including sensors and therapeutic devices. A small MEMS accelerometer may generate five microwatts, for example, which may be sufficient to power a simple sensor.

In some embodiments of the invention, the element may include on-board logic circuitry or a processor. Circuits such as non-clocked logic circuits may be configured to operate with low supply voltages such as the voltages generated by a motion-powered energy source that employs a MEMS accelerometer. In this way, an element may be self-powered. A motion-powered energy source may support a sensor that not only detects or measures a sensed condition, but also processes information as a function of the detection or measurement. A motion-powered energy source may support a therapeutic element that not only delivers therapy, but performs computations pertaining to the mode of therapy delivered.

When an element is powered by a motion-powered energy source, many advantages may result. First, the element would not be as much of a power drain to the IMD. The element could receive some or all of its power from the motion-powered energy source, thereby prolonging the battery life of the IMD. In some embodiments of the invention, the motion-powered energy source may advantageously serve as a backup power supply to a battery, or vice versa. In the event of a loss of power from one supply, the element may use power from the backup power supply.

In addition, the invention presents a small, self-contained, implantable generator that may result in a saving of space. An element powered by a motion-powered energy source may omit an internal battery, allowing the element to be more miniaturized and more versatile. A motion-powered energy source, which may be smaller than a battery, also does not run down like a battery. As long as the motion-powered energy source continues to respond to motion, the motion-powered energy source may generate power for an indefinite period of time. When a motion-powered energy source moves with each beat of a heart, the motion-powered energy source will continue to generate power as long as the heart continues to beat.

A further advantage is that the presence of a motion-powered energy source may reduce or eliminate the need for electrical connections between the IMD and the element. As noted above, it may be unnecessary for the IMD to be electrically connected to the element to supply power to the element. In some embodiments, the power supplied by the motion-powered energy source may be sufficient not only to support sensing or therapeutic functions, but also to support wireless communication between the element and the IMD. In such embodiments, the element may include a transmitter, receiver or transceiver for wireless communication with the IMD or another device, and it may be possible to eliminate the need for any electrical connection between the element and the IMD. With fewer connections, lead construction may be simplified, and the lead and the element may be more robust.

Many embodiments of the invention have been described. Various modifications may be made without departing from the scope of the claims. For example, the motion-powered energy source and element may be combined in a single unit, and need not be physically separated from one another. Sensor 24 and motion-powered energy source 36 depicted in FIG. 2, for example, may be combined in a single integrated circuit. In some embodiments, the element may be a fully self-powered device, such as a self-powered accelerometer.

Although affixing a motion-powered energy source to a heart may cause the motion-powered energy source to generate power as long as the heart beats, the invention is not limited to implantation in or near the heart. Nor is the invention limited to pacemakers such as the device depicted in FIG. 1, but may be practiced with a wide range of implanted diagnostic, therapeutic or monitoring devices. These and other embodiments are within the scope of the following claims.

## Claims

1. A device comprising:
a generator (50, 54) implantable in a human body to convert mechanical energy into electrical energy;
an energy storage device (60) to receive the electrical energy from the generator and to store the electrical energy; and
an element (24) implantable in the human body that receives energy from the energy storage device; **characterised in that** said generator comprises a microelectromechanical systems, i.e. MEMS, accelerometer comprising one or more capacitive elements whose geometry changes in response to movement of an organ of the human body in proximity to which the generator is, in use, implanted, causing a change in the capacitance of the accelerometer.

2. The device of claim 1, wherein the MEMS device comprises at least one of an in-plane overlap MEMS accelerometer, an in-plane gap-closing MEMS accelerometer and an out-of-plane gap-closing MEMS accelerometer.

3. The device of any preceding claim, wherein the element comprises at least one of a pressure sensor, an oxygen sensor, a pH sensor, a glucose sensor, an electrode to deliver a shock to adjacent tissue, a strain gauge, a motion sensor, a contractility sensor, and a controller to control the operation of a medical device.

4. The device of any preceding claim, further comprising an elongated lead body that houses the at least one of the generator, the energy storage device and the element.

5. The device of claim 4, further comprising at least one of a pacemaker, a cardioverter, a defibrillator, a loop recorder, a neurological stimulation device and a drug delivery system coupled to the lead.

6. The device of any preceding claim, further comprising at least one of a transmitter, a receiver and a transceiver to communicate wirelessly with a second device.

## Patentansprüche

1. Vorrichtung mit:
einem in einen menschlichen Körper implantierbaren Generator (50, 54) zur Umwandlung mechanischer Energie in elektrische Energie;
einer Energiespeichervorrichtung (60) zur Aufnahme der elektrischen Energie von dem Generator und zur Speicherung der elektrischen Energie; und
einem in einen menschlichen Körper implantierbaren Element (24) zur Aufnahme von Energie von der Energiespeichervorrichtung; **dadurch gekennzeichnet, dass** der Generator einen Mikroelektromechanik-System- bzw. MEMS-Beschleunigungsmesser mit einem oder mehreren kapazitiven Elementen aufweist, dessen Geometrie sich in Reaktion auf eine Bewegung eines Organs des menschlichen Körpers, in dessen Nähe sich der implantierte Generator befindet, ändert, was eine Änderung in der Kapazität des Beschleunigungsmessers bewirkt.

2. Vorrichtung gemäß Anspruch 1, wobei die MEMS-Vorrichtung wenigstens eine der Komponenten ebener- bzw. plangleicher, überlappender MEMS-Beschleunigungsmesser, plangleicher MEMS-Beschleunigungsmesser für Lückenschluss, MEMS-Beschleunigungsmesser auf versetzten Ebenen für Lückenschluss aufweist.

3. Vorrichtung gemäß einem der vorhergehenden Ansprüche, wobei das Element wenigstens eine der Komponenten Drucksensor, Sauerstoffsensor, pH-Wertsensor, Glukosesensor, Elektrode zur Abgabe eines Schocks an angrenzendes Gewebe, Dehnungsmessstreifen, Bewegungssensor, Kontraktionsfähigkeitssensor, sowie Controller bzw. Schaltelement zum Steuern des Betriebs einer medizinischen Vorrichtung aufweist.

4. Vorrichtung gemäß einem der vorhergehenden Ansprüche, welche ferner einen langgestreckten Leitungskörper aufweist, welcher wenigstens eine der Komponenten Generator, Energiespeichervorrichtung und Element aufweist.

5. Vorrichtung gemäß Anspruch 4, welche ferner wenigstens eine der Komponenten Schrittmacher, Kardioverter, Defibrillator, Endlosaufnahmevorrichtung, neurologischer Stimulator, sowie mit der Leitung verbundenes Wirkstoffabgabesystem aufweist.

6. Vorrichtung gemäß einem der vorhergehenden Ansprüche, welche ferner wenigstens eine der Komponenten Sender, Empfänger, sowie Sender-Empfänger zur drahtlosen Kommunikation mit einer zweiten Vorrichtung aufweist.

## Revendications

1. Dispositif comportant :
un générateur (50, 54) implantable dans un corps humain pour convertir une énergie mécanique en énergie électrique ;
un dispositif de stockage d'énergie (60) pour recevoir l'énergie électrique à partir du générateur et stocker l'énergie électrique ; et
un élément (24) implantable dans le corps humain qui reçoit de l'énergie depuis le dispositif de stockage d'énergie ; **caractérisé en ce que** ledit générateur comporte un accéléromètre de systèmes micro-électromécaniques, c'est-à-dire MEMS, comportant un ou plusieurs éléments capacitifs dont la géométrie change en réponse à un mouvement d'un organe du corps humain à proximité duquel le générateur est, habituellement, implanté, entraînant un changement de la capacité de l'accéléromètre.

2. Dispositif selon la revendication 1, dans lequel le dispositif MEMS comporte au moins l'un parmi un accéléromètre MEMS à chevauchement dans le plan, un accéléromètre MEMS à fermeture d'écartement dans le plan et un accéléromètre MEMS à fermeture d'écartement en dehors du plan.

3. Dispositif selon l'une quelconque des revendications précédentes, dans lequel l'élément comporte au moins l'un parmi un capteur de pression, un capteur d'oxygène, un capteur de pH, un capteur de glucose, une électrode pour délivrer un choc à un tissu adjacent, une jauge de déformation, un capteur de mouvement, un capteur de contractilité, et un contrôleur pour commander le fonctionnement d'un dispositif médical.

4. Dispositif selon l'une quelconque des revendications précédentes, comportant de plus un corps de raccord allongé qui loge le au moins un parmi le générateur, le dispositif de stockage d'énergie et l'élément.

5. Dispositif selon la revendication 4, comportant de plus au moins l'un parmi un stimulateur cardiaque, un cardioverter, un défibrillateur, un enregistreur à boucle, un dispositif de stimulation neurologique et un système d'administration de médicaments couplé au raccord.

6. Dispositif selon l'une quelconque des revendications précédentes, comportant de plus au moins l'un parmi un émetteur, un récepteur et un émetteur-récepteur pour communiquer sans fil avec un second dispositif.
